# EUROPEAN PATENT SPECIFICATION

(11) **EP 1 916 993 B1**
(45) Date of publication and mention of the grant of the patent: **02.11.2016**
(21) Application number: 06802141.9
(22) Date of filing: 23.08.2006
(51) Int. Cl.: A61K 8/365, A61K 8/368, A61K 8/60, A61K 8/66, A61K 8/67, A61K 8/97, A61Q 19/10

(54) **COSMETIC COMPOSITION CONTAINING A PROTEASE ACTIVATOR**
KOSMETISCHE ZUSAMMENSETZUNG MIT EINEM PROTEASE-AKTIVATOR
COMPOSITION COSMETIQUE CONTENANT UN ACTIVATEUR DE PROTEASE

(30) Priority: 26.08.2005 US 711650 P
(43) Date of publication of application: 07.05.2008
(73) Proprietor: ELC Management LLC, New York, NY 10153 (US)
(72) Inventor: GIACOMONI, Paolo, U., Commack, NY 11725 (US); LENTINI, Peter, J., Bellmore, NY 11710 (US); SYED, Ismail, Ronkonkoma, NY 11779 (US); SPARACIO, Rose, Marie, Manorville, NY 11949 (US)
(74) Representative: Hirsch & Associés
(86) International application number: PCT/US2006/032862
(87) International publication number: WO 2007/024915

(56) References cited:
- EP-A1- 1 413 291
- WO-A2-02/060381
- US-A- 5 204 105
- US-A- 5 391 373
- US-A- 5 391 373
- US-A- 5 571 503
- US-A- 5 571 503
- US-A1- 2004 081 681
- US-A1- 2004 081 681
- US-B1- 6 800 292
- DATABASE WPI Week 200162 Thomson Scientific, London, GB; AN 2001-553690 XP002731754, -& JP 2001 220344 A (ICHIMARU PHARCOS INC) 14 August 2001 (2001-08-14)
- DATABASE WPI Week 200308 Thomson Scientific, London, GB; AN 2003-078769 XP002731755, -& JP 2002 234845 A (NARISU KESHOHIN KK) 23 August 2002 (2002-08-23)
- DATABASE WPI Week 200066 Thomson Scientific, London, GB; AN 2000-675245 XP002731756, -& JP 2000 247907 A (KANEBO LTD) 12 September 2000 (2000-09-12)
- DATABASE WPI Week 199844 Thomson Scientific, London, GB; AN 1998-515065 XP002731757, -& JP H10 226633 A (COREANA COSMETICS CO LTD) 25 August 1998 (1998-08-25)

## Description

### Field of the Invention

The present invention discloses a cosmetic composition for application to the skin. In particular, the present invention discloses a cosmetic composition that enhances radiance, glow, smoothness and overall appearance of skin by providing an activator of enzymes resident in the stratum corneum.

### Background of the Invention

The top layer of skin, known as the stratum corneum, is composed primarily of cells that are mainly composed of dehydrated keratin protein. In a normal process called differentiation, epidermal cells are gradually pushed to the surface by underlying cells, where they exfoliate. In abnormal processes, i.e., when these cells do not exfoliate, they accumulate on the surface to form hyperkeratotic tissue causing discomfort. Hyperkeratotic tissue in mammals includes tylomas (calluses and fissures), helomas (corns), keratoses (papules), and dry skin scales and flakes, including dandruff.

Exfoliation is the removal of flaky corneocytes that are ready to detach from the stratum corneum, and so promotes smoother, less flaky skin. Exfoliation improves skin cleansing by helping to mechanically remove dirt, oil and microorganisms from the skin. Other potential health benefits to exfoliation in addition to improved scale (flake) removal and oil removal, as suggested above, are reduction in bacteria on the skin and possibly increased blood flow to the skin due to the mechanical stimulation.

Common methods for managing hyperkeratinized tissue are the application of keratolytic agents such as alpha hydroxy acids (AHA) or salicylic acids in lotion or cream carriers or mechanical scraping to induce exfoliation. However, such keratolytic agents and mechanical scraping can be disadvantageous because they tend to irritate normal epidermis. Moreover, the amount of salicylic acid allowed to be used in cosmetics vary from country to country, thus somewhat limiting the possibility to formulate one product for all the markets for certain consumers, such as those with sensitive skin. Further, salicylic acid has the potential to be irritating but less so than AHAs. Ingredients such as glucosamine or its analogs have been added to salicylic acid to reduce the amount of salicylic acid, however, there remains a need to create compositions that provide satisfactory exfoliation.

It is known that a glucidic purified fraction of chestnut regulates the barrier function of the skin by normalizing the adhesion or desquamation process. This regulation takes place by restructuring epidermic lipids synthesis mechanisms and enhancing epidermal differentiation. Thus, the glucidic purified fraction has been reported to maintain the cutaneous homeostasis and the hydration state of the skin. For example, several documents of the prior art (US2004/081681, US5571503, US5391373 and JP2001220344) disclose compositions suitable for exfoliation comprising a chesnut extract and various compounds such as papain carbomer, retinyl palmitate or resorcinol. However, it has not heretofore been known to combine a chestnut extract with the other components of the present invention to exfoliate the skin by modulating the enzymatic function in the stratum corneum.

Therefore, there is a need for a composition and method of exfoliating that avoids irritation or other undesired secondary effects.

### Summary of the Invention

The present invention discloses an exfoliation composition comprising a protease activator comprising at least one chestnut extract, at least one exfoliator and a cosmetically acceptable vehicle.

The present invention further discloses a method of exfoliating human skin comprising the step of combining a protease activator and an exfoliator to enhance the exfoliating activity of the protease activator such as chestnut extract, and topically applying to the skin a composition comprising the protease activator and the exfoliator.

### Detailed Description

In the stratum corneum, corneocytes are held together by proteic bonds as well as by saccharidic bonds. While not wishing to be bound by any theories, it is believed that the use of acidic creams and lotions provoke the breaking of these bonds, but with undesirable side effects as described above. To break the intercorneocytary bonds without acids it is believed that one must act on both types of bonds. The present composition discloses a protease activator with an exfoliator to surprisingly enhance exfoliation activity of the protease activator. Again, not wishing to be bound by any theories, it is believed that the combination in the present inventive composition enhances the activity of resident proteases such as trypsin or chymotrypsin to enable displacement of the saccharidic bonds while breaking the proteic bonds. It has further been surprisingly discovered that the inherent exfoliating effect of a protease activator, which otherwise pales in comparison to known exfoliating agents that act according to a separate mechanism, is enhanced in the presence of known exfoliating agents that act by a separate mechanism.

The present invention thus relates to an exfoliation composition comprising a protease activator that is at least one chestnut extract of *Castanea sativa* of the family Fagaceae, wherein the chestnut extract is selected from the group consisting of polysaccharides, oligosaccharides, ceramides, sphingosines, phytosines and lysine; at least one exfoliator selected from the group consisting of beta hydroxy acids, alpha hydroxy acids, N-acetyl-D-glucosamine, proteolytic enzymes, and retinoic acid; and a cosmetically acceptable vehicle.

Protease activators alone have shown little inherent exfoliating effect after application onto the skin, as shown in Table 2 below. Moreover, as further shown in Table 2 below, compositions comprising a combination of known exfoliators with a protease activator have shown improvement in overall exfoliating effect upon application onto the skin. The combination of a protease activator and an exfoliator surprisingly renders the protease activator having a greater exfoliating effect than the protease activator demonstrates alone.

The first essential ingredient of the exfoliation composition as disclosed is a protease activator comprising at least one chestnut extract. The protease activator is selected from the group consisting of polysaccharides (e.g., oligosaccharide), ceramides, sphingosines, phytosines and lysine. Different forms of the chestnut extract may be used, including water and alcohol derived extracts, and glucidic purified and hydrolyzed based extracts. Preferably, a glucidic purified fraction of chestnut is used in the present inventive composition (commercially available as Recoverine® from Silab). Active components of chestnut extract include but are not limited to rhamnogalacturonans and uronic acids. Other actives that act as protease activators that are commercially available, include but are not limited to, for example, Exfolactive® from Silab, an extract of oligosaccharides from nopal (prickly pear).

The protease activator is used in an amount dictated by the reaction equilibrium constant (Km). The protease activator, by weight of the dry active components, may be used from an amount of from about 0.001 to 10% , preferably from about 0.1 to 8% , and most preferably from about 0.4 to 6%.

The second essential ingredient as disclosed is an exfoliator. The exfoliator is an ingredient known to persons of ordinary skill in the art to aid in exfoliation of the skin. Exfoliators of the present invention are beta hydroxy acids, alpha hydroxy acids, N-acetyl-D-glucosamine, proteolytic enzymes, and retinoic acid. Beta hydroxy acids and alpha hydroxy acids may be selected from the group consisting of glycolic acid, lactic acid, salicylic acid, methyllactic acid, citric acid, malic acid, tartaric acid, saccharic acid, glucoheptonic acid, galacturonic acid, glucuronic acid, mandelic acid, mucic acid, pyruvic acid, and tartronic acid. Proteolytic enzymes may be selected from the group consisting of papain, pepsin, peptidase, trypsin and enterokinase. Particularly preferred in the present invention is N-acetyl-D-glucosamine as the exfoliator or N-acetyl-D-glucosamine and beta hydroxy acid in combination as the exfoliator in combination with the protease inhibitor. More particularly preferred, the beta hydroxy acid is salicylic acid.

The exfoliator may be used in an amount from about 0.01 to 20%, preferably from about 0.1 to 5%, and most preferably from about 0.1 to 2.0%, although amounts may vary depending upon the exfoliator chosen for the composition. For example, when the exfoliator is N-acetyl-D-glucosamine, the amount useful in the present invention is about 0.01 to 2.0%, preferably about 0.05 to 1.0%, and most preferably about 0.1 to 0.5%; and when the exfoliator is salicylic acid, the amount useful in the present invention is 0.01 to 5.0%, preferably 0.05 to 2.0%, and most preferably 0.1 to 1.0%.

The present composition can be used in virtually any type of topically useful vehicle, in amounts capable of enhancing the exfoliating effect upon application onto the skin. The carriers will be those that are cosmetically acceptable, that is, a vehicle for cosmetic use, intended for application to skin, which vehicle delivers the active components to the intended target and which will not cause harm to the average human when applied to the surface intended to be treated. As used herein, "cosmetic" will be understood to encompass human cosmetics with which the active component is compatible, e.g., a gel, a cream, a lotion, an ointment, a spray, a solid stick, a powder, a suspension, a dispersion and the like. Techniques for formulation of various types of vehicles are well known to those skilled in the art, and can be found, for example, in Chemistry and Technology of the Cosmetics and Toiletries Industry, Williams and Schmitt, eds., Blackie Academic and Professional, Second Edition, 1996 Harry's Cosmeticology, Eighth Edition, M. Reiger, ed. (2000), and Remington: The Science and Practice of Pharmacy, Twentieth Edition, A. Gennaro, ed. (2003), the contents of each of these being incorporated by reference herein.

Any typical composition that is useful for topical delivery, for example, aqueous dispersions, anhydrous compositions, emulsions (silicone/oil-in-water, water-in-oil/silicone) multiple emulsions, microemulsions, nanoemulsions), can be employed, provided the components are compatible with the essential ingredients of the present invention. For example, some silicone emulsifiers may not be compatible with certain ingredients, in which case a non-silicone based emulsion composition should be used. Such a need can be determined through routine experimentation.

### Optional Components

Additional components include, but are not limited to antioxidants (such as BHT); chelating agents (such as disodium EDTA); preservatives (such as methyl paraben); fragrances (such as pinene); humectants (such as glycerine); moisturizing agents (such as cholesterol, butylenes glycol); vitamins (such as tocopherol); sunscreens (such as octyl methoxycinnamate, titanium dioxide, zinc oxide, camphor derivatives, cinnamates, salicylates, benzophenones, triazines, PABA derivatives, diphenylacrylate derivatives, and dibenzoylmethane derivatives) and the like.

The compositions can also encompass one or more additional active components, and as such can be either cosmetic or pharmaceutical compositions in addition to exfoliating cosmetics, such as anti-aging and concentrations may be determined by one skilled in the art to determine effectiveness of product as discussed in the present invention. Such additional active components are added at concentrations such that they do not interfere with the exfoliating properties of the inventive composition and do not contribute to negative side effects such as irritation. Examples of useful actives include, but are not limited to, those that improve or eradicate age spots, keratoses and wrinkles, analgesics, anesthetics, anti-acne agents, antibacterials, antiyeast agents, antifungal agents, antiviral agents, antidermatitis agents, antipruritic agents, antiemetics, antihyperkeratolytic agents, anti-dry skin agents, antipsoriatic agents, antiseborrheic agents, antiaging agents, antiwrinkle agents, antihistamine agents, wound-healing agents, vitamins, corticosteroids, tanning agents or hormones. More specific examples of useful active agents include tocopherol and esters and amide derivatives thereof; shark cartilage; milk proteins; DHEA and derivatives thereof; topical cardiovascular agents; clotrimazole, ketoconazole, miconozole, griseofulvin, hydroxyzine, diphenhydramine, pramoxine, lidocaine, procaine, mepivacaine, monobenzone, erythromycin, tetracycline, clindamycin, meclocyline, hydroquinone, minocycline, naproxen, ibuprofen, theophylline, cromolyn, albuterol, hydrocortisone, hydrocortisone 21-acetate, hydrocortisone 17-valerate, hydrocortisone 17-butyrate, betamethasone valerate, betamethasone diproprionate, triaminolone acetonide, fluocinonide, clobetasol, proprionate, benzoyl peroxide, crotamiton, propranol, promethazine, and mixtures thereof.

Particularly preferred embodiments of the present formulations are skin care formulas used as exfoliating compositions.

The compositions are formulated with the water soluble ingredients mixed together, the oil soluble ingredients mixed together, and adding the oil mixture to the water mixture or the water mixture to the oil mixture, depending on the type of emulsion desired.

### Methods of Use

The present compositions are particularly useful as products for methods of enhancing exfoliation on human skin (particularly enhancing the uniformity of exfoliation on the face) and improving the appearance of skin with cosmetic compositions as well as diminishing the appearance of lines and wrinkles on the skin. The methods of the present invention disclose the enhancement of the exfoliating activity of the protease activator. The present invention thus relates to a method of exfoliating human skin comprising applying the composition comprising a protease activator that is at least one chestnut extract of *Castanea sativa* of the family Fagaceae, wherein the chestnut extract is selected from the group consisting of polysaccharides, oligosaccharides, ceramides, sphingosines, phytosines and lysine; at least one exfoliator selected from the group consisting of beta hydroxy acids, alpha hydroxy acids, N-acetyl-D-glucosamine, proteolytic enzymes, and retinoic acid; and a cosmetically acceptable vehicle More preferably, the chestnut extract is a glucidic purified fraction of chestnut or hydrolyzed chestnut extract. Preferably, the exfoliant is N-acetyl-D-glucosamine and a beta hydroxy acid. More preferably, the beta hydroxy acid is salicylic acid.

Such methods comprise administering or topically applying to the skin a safe and effective amount of the composition of the present invention. The amounts of the components in the compositions will vary depending upon the level of exfoliation desired and the individual's skin type.

It is suggested as an example that topical application range from about once per month to about twice daily, preferably from about once every week to about once every other day, most preferably about once per day. Preferably, the composition is applied in an amount of from 2 mg/cm2 to 100 mg/cm2. The amount of composition applied to the skin will vary depending upon coverage desired.

The following examples further illustrate the invention, but the invention is not limited thereto.

### Example 1

Compositions comprising the inventive composition including chestnut extract (Recoverine®) alone is compared with known exfoliators salicylic acid and N-acetyl glucosamine (NADG) for exfoliation in a 24 hour period.

Twenty women apply products to the face twice, once in the morning and once in the evening. One formulation is applied to the right half of the face, while a second formulation is applied to the left half of the face.

The next morning, skin exfoliation is assessed via D-Squames (CuDerm Corporation, Dallas, Texas). These 22mm diameter, clear coated discs have a homogenous layer of adhesive which removes superficial corneocytes from the stratum corneum. Two D-Squames are taken from each site at each visit and mounted on clear microscope slides. The slides are then illuminated in a light box and viewed by a CCD video camera. The video images of the samples are captured by a personal computer and processed with an image analysis program, Optimas 6.51. The integrated optical density (IOD) is evaluated for skin exfoliation. As the IOD decreases, less lose cells and scales are being removed, and hence, the smoother the skin.

The base formula for the tests conducted is provided below in Table 1 and the results are provided in Table 2 below. The amount of water is adjusted to accommodate the addition of the Chestnut Extract and exfoliators tested in the present experiment. In addition to the essential ingredients, the above composition includes additional ingredients such as a preservative, moisturizing agents and thickener.

**Table 1**

| INGREDIENT | AMOUNT |
|---|---|
| Water | 84.85% |
| Lexol GT-865/Liponate G | 8.50% |
| Simulgel 600 (Acrylamide/Sodium Acryloyldimethyl Taurate Copolymer & Isohexadecane & Polysorbate 80) | 1.00% |
| 1,3 Butylene glycol | 2.00% |
| Phenoxetol (Phenoxyethanol) | 0.70% |
| Sensiva SC 50 (2-Ethylhexyl Glycerylether) | 0.50% |
| Caprylyl Glycol (Diocide®) | 0.70% |
| Vanzan® NF-F (xanthan gum) | 0.50% |
| EDTA BD/ NA2 (Disodium Ethylendiaminetetraacetic acid salt) | 0.050% |
| | |
| TOTAL | 98.80% |

### Results

**Table 2**

| | Chestnut Extract (%) | N-acetyl-D-glucosamine (%) | Salisomes (%) (containing 10% salicylic acid) | Exfoliation Parameter improvement (%) |
|---|---|---|---|---|
| G1 | 0 | 0.2 | 0 | 10 |
| Y1 | 0 | 0.2 | 5.0 | 15 |
| G2 | 1 | 0.2 | 0 | 12 |
| O | 4 | 0.2 | 0 | 16 |
| Y2 | 4 | 0.2 | 5.0 | 20 |
| | 5 | 0 | 5.0 | 14 |
| G3 | 5 | 0 | 0 | 2 |

As seen from the tables above, a composition comprising Chestnut Extract (Recoverine®) in an amount of 5% alone only results in a 2% increase in exfoliation parameter improvement (%) based on IOD measurements as described above in comparison to a control with no exfoliator (see row G3), while a composition containing the known exfoliator N-acetyl-D-glucosamine (NADG) in an amount of 0.2% increases exfoliation parameter improvement to 10% over the control (see row G1). Therefore, Recoverine® is considered relatively less effective than NADG as an exfoliating agent.

Surprisingly, however, a composition containing a combination of 0.2% NADG and Recovering® in an amount of 1% and 4% renders an increase in exfoliation parameter improvement capacity over the 0.2% NADG only composition from 10% to 12%, and 10% to 16%, respectively (see rows G2 and O each in comparison with row G1). In conclusion, Recoverine®, considered to be a poor exfoliator as compared with NADG, when added to a known exfoliator, unexpectedly demonstrates a greater exfoliation parameter improvement capacity. When analyzed alone, 5% Recoverine® yields a 2% exfoliation parameter improvement over a control (see row G3); whereas, when analyzed in combination with 0.2% NADG, 1% Recoverine® yields a 2% exfoliation parameter improvement over 0.2% NADG alone (12%-10%=2%; see rows G2 and G1), and 4% Recoverine® yields a 6% exfoliation parameter improvement over 0.2% NADG alone (16%-10%=6%; see rows O and G1). This is surprising because combining 4% Recoverine® with 0.2% NADG produces a 3-fold increase (2% alone as shown in row G3 versus 6% in combination with 0.2% NADG as shown in rows O and G1; 2%*3=6%) in exfoliation parameter improvement as compared with Recoverine® alone.

With respect to combining 1% Recoverine® in combination with 0.2% NADG, the result is equally if not more surprising because 1% Recoverine® in combination with 0.2% NADG exhibits activity with respect to exfoliation comparable to 5% Recoverine® alone (i.e., 5% Recoverine® alone renders a 2% exfoliation parameter improvement as shown in row G3 as compared with 1% Recoverine® that also renders a 2% exfoliation parameter improvement when combined with 0.2% NADG as shown in rows G2 and G1). Therefore, 1% Recoverine® in combination with 0.2% NADG is 5 times as effective as 5% Recoverine® alone.

A similar effect found with Recoverine® in combination with 0.2% NADG is found with Recoverine® in combination with 0.2% NADG and salicylic acid present in the amount of 5% Salisomes® containing 10% salicylic acid. When 4% Recoverine® is added to a combination of 0.2% NADG and salicylic acid (5% Salisomes® containing 10% salicylic acid) the exfoliation parameter improvement increases from 15% without Recoverine® (see row Y1) to 20% with Recoverine® (see row Y2), a 5% increase. This increase is again better than the exfoliation parameter improvement exhibited by 5% Recoverine® alone (see row G3). In fact, 4% Recoverine® in combination with 0.2% NADG and 5% Salisomes® containing 10% salicylic acid is at least 3 times as effective as 5% Recoverine® alone in the exfoliation parameter improvement (4% Recoverine® added to a combination of 0.2% NADG and 5% Salisomes® containing 10% salicylic acid produces a 5% increase in the exfoliation parameter improvement (see rows Y1 and Y2) compared to 5% Recoverine® alone that produces a 2% increase in the exfoliation parameter improvement (see row G3)). Based on the data above, it would take about 12.5% Recoverine® alone to yield a 5% increase in the exfoliation parameter compared to the 4% Recoverine® in combination with 0.2% NADG and 5% Salisomes® containing 10% salicylic acid (12.5% divided by 4% is 3.125%, and therefore, 4% Recoverine® in combination with 0.2% NADG and 5% Salisomes® containing 10% salicylic acid is at least 3 times as effective as 5% Recoverine® alone.) Therefore, Recoverine® is enhanced as an exfoliating agent when combined with a known exfoliating agent.

It should be understood that the specific forms of the invention herein illustrated and described are intended to be representative only. Changes, including but not limited to those suggested in this specification, may be made in the illustrated embodiments without departing from the clear teachings of the disclosure. Accordingly, reference should be made to the following appended claims in determining the full scope of the invention.

## Claims

1. An exfoliation composition comprising:
a protease activator that is at least one chestnut extract of *Castanea sativa* of the family Fagaceae, wherein the chestnut extract is selected from the group consisting of polysaccharides, oligosaccharides, ceramides, sphingosines, phytosines and lysine;
at least one exfoliator selected from the group consisting of beta hydroxy acids, alpha hydroxy acids, N-acetyl-D-glucosamine, proteolytic enzymes, and retinoic acid; and
a cosmetically acceptable vehicle.

2. The composition of claim 1 wherein the chestnut extract is a glucidic purified fraction of chestnut or a hydrolyzed chestnut.

3. The composition of claim 2 wherein the chestnut extract is a glucidic purified fraction of chestnut.

4. The composition of claim 1 wherein the beta hydroxy acids and alpha hydroxy acids are selected from the group consisting of glycolic acid, lactic acid, salicylic acid, methyllactic acid, citric acid, malic acid, tartaric acid, saccharic acid, glucoheptonic acid, galacturonic acid, glucuronic acid, mandelic acid, mucic acid, pyruvic acid and tartronic acid.

5. The composition of claim 1 wherein the proteolytic enzymes are selected from the group consisting of papain, pepsin, peptidase, trypsin, and enterokinase.

6. The composition of claim 1 wherein the exfoliator is a combination of beta hydroxy acid and N-acetyl-D-glucosamine.

7. The composition of claim 6 wherein the exfoliator is a combination of salicylic acid and N-acetyl-D-glucosamine.

8. The composition of claim 1 wherein the exfoliator is N-acetyl-D-glucosamine.

9. A method of exfoliating human skin comprising applying the composition of claim 1 to the skin.

10. The method of claim 9 wherein the chestnut extract is a glucidic purified fraction of chestnut or hydrolyzed chestnut extract.

11. The method of claim 9 wherein the exfoliator is selected from the group consisting of beta hydroxy acid, alpha hydroxy acids, N-acetyl-D-glucosamine, and proteolytic enzymes.

12. The method of claim 11 wherein the beta hydroxy acids and alpha hydroxy acids are selected from the group consisting of glycolic acid, lactic acid, salicylic acid, retinoic acid, methyllactic acid, citric acid, malic acid, tartaric acid, saccharic acid, glucoheptonic acid, galacturonic acid, glucuronic acid, mandelic acid, mucic acid, pyruvic acid and tartronic acid

13. The method of claim 11 wherein the proteolytic enzymes are selected from the group consisting of papain, pepsin, peptidase, trypsin, and enterokinase.

## Patentansprüche

1. Peeling-Zusammensetzung umfassend:
- einen Proteaseaktivator, der mindestens ein Kastanienextrakt von Castanea sativa der Familie Fagaceae ist, wobei das Kastanienextrakt aus der Gruppe ausgewählt wird bestehend aus Polysacchariden, Oligosacchariden, Ceramiden, Sphingosinen, Phytosinen und Lysin;
- mindestens ein Peeling-Mittel ausgewählt aus der Gruppe bestehend aus Beta-Hydroxysäuren, Alpha-Hydroxysäuren, N-Acetyl-D-glucosamin, proteolytischen Enzymen und Retinsäure; und
- einem kosmetisch akzeptablen Vehikel.

2. Zusammensetzung nach Anspruch 1, wobei das Kastanienextrakt eine glucidische gereinigte Fraktion von Kastanie oder einer hydrolysierten Kastanie ist.

3. Zusammensetzung nach Anspruch 2, wobei das Kastanienextrakt eine glucidische, gereinigte Fraktion von Kastanie ist.

4. Zusammensetzung nach Anspruch 1, wobei die Beta-Hydroxysäuren und Alpha-Hydroxysäuren aus der Gruppe ausgewählt sind bestehend aus Glycolsäure, Milchsäure, Salicylsäure, Methylmilchsäure, Zitronensäure, Apfelsäure, Weinsäure, Zuckersäure, Glucoheptonsäure, Galacturonsäure, Glucuronsäure, Mandelsäure, Schleimsäure, Brenztraubensäure und Tartronsäure.

5. Zusammensetzung nach Anspruch 1, wobei die proteolytischen Enzyme aus der Gruppe ausgewählt sind bestehend aus Papain, Pepsin, Peptidase, Trypsin und Enterokinase.

6. Zusammensetzung nach Anspruch 1, wobei das Peeling-Mittel eine Kombination von Beta-Hydroxysäure und N-Acetyl-D-glucosamin ist.

7. Zusammensetzung nach Anspruch 6, wobei das Peeling-Mittel eine Kombination von Salicylsäure und N-Acetyl-D-glucosamin ist.

8. Zusammensetzung nach Anspruch 1, wobei das Peeling-Mittel N-Acetyl-D-glucosamin ist.

9. Verfahren für das Peeling menschlicher Haut, umfassend das Aufbringen der Zusammensetzung nach Anspruch 1 auf die Haut.

10. Verfahren nach Anspruch 9, wobei das Kastanienextrakt eine glucidische, gereinigte Fraktion von Kastanie oder hydrolysiertes Kastanienextrakt ist.

11. Verfahren nach Anspruch 9, wobei das Peeling-Mittel aus der Gruppe ausgewählt ist bestehend aus Beta-Hydroxysäure, Alpha-Hydroxysäuren, N-Acetyl-D-glucosamin und proteolytischen Enzymen.

12. Verfahren nach Anspruch 11, wobei die Beta-Hydroxysäuren und Alpha-Hydroxysäuren aus der Gruppe ausgewählt sind bestehend aus Glycolsäure, Milchsäure, Salicylsäure, Retinsäure, Methylmilchsäure, Zitronensäure, Apfelsäure, Weinsäure, Zuckersäure Glucoheptonsäure, Galacturonsäure, Glucuronsäure, Mandelsäure, Schleimsäure, Brenztraubensäure und Tartronsäure.

13. Verfahren nach Anspruch 11, wobei die proteolytischen Enzyme aus der Gruppe ausgewählt sind bestehend aus Papain, Pepsin, Peptidase, Trypsin und Enterokinase.

## Revendications

1. Composition exfoliante comprenant :
un activateur de protéase qui est au moins un extrait de châtaigne de *Castanea sativa* de la famille des Fagaceae, dans laquelle l'extrait de châtaigne est choisi dans le groupe constitué par les polysaccharides, les oligosaccharides, les céramides, les sphingosines, les phytosines et la lysine ;
au moins un agent exfoliant choisi dans le groupe constitué par les acides bêta-hydroxy, les acides alpha-hydroxy, la N-acétyl-D-glucosamine, les enzymes protéolytiques, et l'acide rétinoïque ; et
un véhicule acceptable sur le plan cosmétique.

2. Composition selon la revendication 1 dans laquelle l'extrait de châtaigne est une fraction glucidique purifiée de châtaigne ou un extrait de châtaigne hydrolysé.

3. Composition selon la revendication 2 dans laquelle l'extrait de châtaigne est une fraction glucidique purifiée de châtaigne.

4. Composition selon la revendication 1 dans laquelle les acides bêta-hydroxy et les acides alpha-hydroxy sont choisis dans le groupe constitué par l'acide glycolique, l'acide lactique, l'acide salicylique, l'acide méthyllactique, l'acide citrique, l'acide malique, l'acide tartrique, l'acide saccharique, l'acide glucoheptonique, l'acide galacturonique, l'acide glucuronique, l'acide mandélique, l'acide mucique, l'acide pyruvique et l'acide tartronique.

5. Composition selon la revendication 1 dans laquelle les enzymes protéolytiques sont choisies dans le groupe constitué par la papaïne, la pepsine, la peptidase, la trypsine, et l'entérokinase.

6. Composition selon la revendication 1 dans laquelle l'agent exfoliant est une combinaison d'acide bêta-hydroxy et de N-acétyl-D-glucosamine.

7. Composition selon la revendication 6 dans laquelle l'agent exfoliant est une combinaison d'acide salicylique et de N-acétyl-D-glucosamine.

8. Composition selon la revendication 1 dans laquelle l'agent exfoliant est la N-acétyl-D-glucosamine.

9. Méthode d'exfoliation de la peau humaine comprenant l'application de la composition selon la revendication 1 sur la peau.

10. Méthode selon la revendication 9 dans laquelle l'extrait de châtaigne est une fraction glucidique purifiée de châtaigne ou un extrait de châtaigne hydrolysé.

11. Méthode selon la revendication 9 dans laquelle l'agent exfoliant est choisi dans le groupe constitué par l'acide bêta-hydroxy, les acides alpha-hydroxy, la N-acétyl-D-glucosamine, et les enzymes protéolytiques.

12. Méthode selon la revendication 11 dans laquelle les acides bêta-hydroxy et les acides alpha-hydroxy sont choisis dans le groupe constitué par l'acide glycolique, l'acide lactique, l'acide salicylique, l'acide rétinoïque, l'acide méthyl-lactique, l'acide citrique, l'acide malique, l'acide tartrique, l'acide saccharique, l'acide glucoheptonique, l'acide galacturonique, l'acide glucuronique, l'acide mandélique, l'acide mucique, l'acide pyruvique et l'acide tartronique.

13. Méthode selon la revendication 11 dans laquelle les enzymes protéolytiques sont choisies dans le groupe constitué par la papaïne, la pepsine, la peptidase, la trypsine, et l'entérokinase.
